# EUROPEAN PATENT SPECIFICATION

(11) **EP 1 829 532 B1**
(45) Date of publication and mention of the grant of the patent: **07.05.2008**
(21) Application number: 06005326.1
(22) Date of filing: 15.03.2006
(51) Int. Cl.: A61K 9/20, A61K 38/11

(54) **Stable solid dosage form comprising desmopressin**
Stabile Desmopressinformulierung in fester Form
Forme posologique solide stable de la desmopressine

(30) Priority: 02.03.2006 SE 0600482
(43) Date of publication of application: 05.09.2007
(73) Proprietor: Ferring International Center S.A., 1162 Saint-Prex (CH)
(72) Inventor: Aston, Christina Ulrika Jonius, 21611 Limhamn (SE); Olsson, Lars-Erik, 21567 Malmö (SE); Nilsson, Lars Anders Ragnar, 20065 Lund (SE)
(74) Representative: HOFFMANN EITLE

(56) References cited:
- EP-A- 1 530 967
- EP-A- 1 550 439
- WO-A-03/094886
- US-A1- 2004 001 887

## Description

The present invention relates to a novel pharmaceutical composition as a solid dosage form comprising desmopressin as a therapeutically active ingredient.

### Background

Desmopressin, also known as dDAVP, is the therapeutically active ingredient (as its acetate salt) in the pharmaceutical product Minirin®, which is marketed *inter alia* as a nasal spray and a tablet formulation. Desmopressin is primarily used in the treatment of primary nocturnal enuresis, i.e. bedwetting, in children, but it is approved also for the treatment of nocturia and diabetes insipidus. The tablet formulation of desmopressin has been marketed since 1987, and several different tablet formulations are authorised for marketing.

The desmopressin tablet formulation is preferably manufactured by compression of a suitable granulate, where the granulate is composed of the required constituents as a mixture of aggregated solid particles. Typical such particles are the therapeutically active ingredient, various excipients, disintegrating agents, lubricants and binders, optionally together e.g. with flavoring agent, preservative and/or colorant. For a comprehensive review of pharmaceutical tablet manufacturing, see "Tableting" (by N.A. Armstrong) in "Pharmaceutics - The science of dosage form design", pp 647-668; Ed. M.E. Aulton, Churchill Livingstone, Edinburgh, London, Melbourne and New York, 1988.

WO 85/02119 A1, WO 95/25534 A1, EP 1 500 390 A1, EP 1 530 967 A1 and EP 1 550 439 A1 disclose exemplary tablet formulations of desmopressin, including their manufacturing.

The prior art establishes that the desmopressin molecule, which is a nonapeptide having a disulfide bond, is sensitive to degradation. Keeping desmopressin stable *per se* under various conditions, including long term shelf storage, is a recurring problem addressed over the years. Desmopressin tablet formulations with market authorisation typically have a shelf life of from 12 to 24 months.

The most common desmopressin tablet consists of desmopressin acetate as active ingredient together with potato starch and lactose as excipients, and a suitable amount of binder(s) and lubricant, respectively. It may also contain a preservative and/or corn starch instead of potato starch.

WO 03/094886 describes pharmaceutical formulations comprising desmopressin and an open matrix network which is an inert water-soluble or water-dispersible carrier material.

US 2004/0001887 describes a bioadhesive controlled extended release progressive hydration composition wherein the active ingredient may be protected from water or the surrounding environment, thereby protecting it from metabolism or from other degradation caused by moisture, enzymes or pH effects, and making it bioavailable only at a controlled rate.

It is an objective of the present invention to provide a means for improving the storage stability of desmopressin in tablet formulations.

### Disclosure of the Invention

It has now been discovered that the presence of residual oxidising agent is linked to the degradation rate of desmopressin during shelf storage. In other words careful control and suppression of the level of residual oxidising agent(s) has been identified as a means for improving the shelf life of desmopressin in tablet formulations.

More specifically, the present invention relates to a pharmaceutical composition as a solid dosage form comprising desmopressin, or a pharmaceutically acceptable salt thereof, as a therapeutically active ingredient together with a pharmaceutically acceptable excipient, diluent or carrier, or mixture thereof, wherein said pharmaceutical composition comprises silica and starch and wherein the level of oxidising agent is equal to or less than 15 parts per million by weight of said pharmaceutical composition.

Part by weight relates to the resulting part of the weight of the final pharmaceutical composition.

In many cases the terms excipient, diluent and carrier can be used interchangeably, and they may even refer to one and the same substance, or to a mixture of similar such substances. The proper use and understanding of these terms is self-explanatory and lies well within the ability of a person skilled in the art of pharmaceutical formulation.

In a preferred embodiment, the level of oxidising agent in said pharmaceutical composition is from 0.01 to less than 5 parts per million by weight of said pharmaceutical composition. More preferably said level is from 0.01 to equal to or less than 3 parts per million. Even more preferably said level is from 0.01 to equal to or less than 1 part per million.

Said oxidising agent may be an organic or inorganic peroxide, or a mixture of peroxides. It is usually hydrogen peroxide, or its content is expressed as hydrogen peroxide equivalents in parts per million by weight.

The present pharmaceutical composition preferably contains silica in an amount of from 0.1 to 1.0, and more preferably from 0.2 to 0.5, percent by weight of said pharmaceutical composition. The expression silica refers to SiO₂, silicon dioxide, in either of its forms, e.g. Aerosil® (marketed by Degussa, Germany) as colloidal silicon dioxide. A general reference on silica and use thereof in pharmaceutical compositions is *"*Handbook of Pharmaceutical Excipients"; Ed. A. H. Kibbe, 3.sup.rd Ed., American Pharmaceutical Association, USA and Pharmaceutical Press UK, 2000, pp 143-145.

The present pharmaceutical composition is preferably composed of a compressed granulate. A granulate suitable for compression into tablets typically has an average granulate size of at least about 100 µm. Discrete granules with a size above 2 mm are usually not employed in a subsequent compression step.

A granulate suitable for compression is suitably prepared by mixing a granulation liquid of solvent, preferably also containing a binder, with solid particle components whereby the latter aggregate to form larger particles, i.e. the desired granulate. Said solvent is preferably selected from water and a mixture of water and an alcohol, preferably ethanol. A water/ethanol 1:3 mixture is typically used, albeit many other combinations are possible.

The present pharmaceutical formulation can be prepared by using conventional equipment. As non-limiting examples mention can be made of the following equipment for granulation: directly heated fluidised solid beds e.g. provided by GEA/Collette NV, BE (UltimaPro^{™} series), Huettlin GmbH, DE (HDG series), Diosna Dierks & Soehne GmbH, DE (VAC series), Fluid Air Inc., US (Magnaflo® series) and Vector Corp., US (GMX series); indirect conduction moving solids bed, including paddle systems, rotary systems and agitation systems, which are e.g. provided by Jaygo Inc., US (JRB and Novamix series), Paul O. Abb Inc., US (Rota-Cone, Rota-U, Rota Blade, Cylindrical Ribbon/Paddle, Plow and Sigma-blade series), Forberg A/S, No (Forberg II series), Gemco Inc. US (D/3 Double Cone, V-Shape and Slant-Cone series), LittlefordDay Inc., US (Double Arm, Day Nauta and Daymax series), Patterson-Kelly, Harsco Corp., US (P-K solids Processor® series), Diosna as above (CCS and VAC series), Romaco Zanchetta SpA, IT (Roto E, Roto D and Roto P series) and L. B. Bohle Maschinen und Verfahren GmbH, DE (Granumator GMA and Vagumator VMA series). The aforementioned equipment in general also provides drying of the prepared granules.

The pharmaceutical composition according to the present invention may optionally comprise at least one additive selected from a disintegrating agent, binder, lubricant, flavoring agent, preservative, colorant and a mixture thereof. Where considered suitable also other additives may be included. Representative examples of disintegrating agents, binders (e.g. Kollidon® 25, BASF), flavoring agents, preservatives and colorants, and suitable mixtures thereof, as well as any other conventional additive that may be considered by a person skilled in the art practising the present invention, can be found in *"*Handbook of Pharmaceutical Excipients"; Ed. A. H. Kibbe, 3.sup.rd Ed., American Pharmaceutical Association, USA and Pharmaceutical Press UK, 2000. As an example, also applicable in the practising of the present invention, a typical amount of binder is in the order of from 1 to 6 percent by weight of the pharmaceutical composition.

Said lubricant is typically selected from a group consisting of stearic acid, salts or esters of stearic acid, hydrogenated vegetable oils, magnesium oxide, polyethylene glycol, sodium lauryl sulphate and talc, and mixtures thereof. Preferably said lubricant is selected from magnesium stearate, calcium stearate, zinc stearate, glyceryl palmitostearate and sodium stearyl fumarate, and mixtures thereof. Magnesium stearate is the most preferred alternative.

As used herein, the expression oligosaccharide relates to a chain, with any degree of branching, of from three to ten monosaccharide units linked via glycoside bonds. Accordingly, as used herein, the expression polysaccharide relates to a chain, with any degree of branching, of at least eleven monosaccharide units linked via glycoside bonds. Synthetically modified derivatives and analogues of naturally occurring saccharides are also possible to use in the practising of the present invention.

In a particularly preferred embodiment at least one of said excipient, diluent and carrier is a substance selected from a monosaccharide, disaccharide, oligosaccharide and a polysaccharide. The most preferred pharmaceutical composition contains both disaccharide and polysaccharide. The weight ratio between said disaccharide and polysaccharide is typically from 100:1 to 1:100, preferably from 10:1 to 1:10, and more preferably from 2:1 to 1:2.

The disaccharide is preferably lactose, such as lactose-α-monohydrate. As exemplary lactose of pharmaceutical suitability mention can be made of Pharmatose®, such as the 150M, DCL 11, DCL 15, DCL 21 and DCL 40 series marketed by DMV (the Netherlands), and Tablettose®, such as the 70, 80 and 100 series marketed by Meggle AG (Germany).

The starch is selected from corn (maize), wheat and potato starch, where potato starch is preferred. It deserves mentioning that significantly varying levels of oxidising agent, usually hydrogen peroxide, are surprisingly most prevalent for the commercial potato starch. It is therefore in association with potato starch that the stability concerns of desmopressin are most pronounced. Exemplary potato starches are Pharma M20, Pharma M14 (provided by KMC, Denmark), and AmylSolVät (provided by Lyckeby Stärkelse AB, Sweden). In one embodiment, the starch used contains oxidising agent, usually H₂O₂, at a level of equal to or less than 40 parts per million by weight, more preferably from 0.03 to equal to or less than 8 parts per million, even more preferably from 0.03 to equal to or less than 3 parts per million.

The total combined amount of said excipient, diluent and carrier is usually from 5 to 98, preferably from 50 to 98, percent by weight of the pharmaceutical composition, the balance up to 100% being desmopressin, or a salt thereof, optionally together with the aforementioned additives. The latter are preferably binder and lubricant, respectively.

The present pharmaceutical composition is preferably a perorally available tablet. As an alternative the tablet may be adapted for oral, including buccal and/or sublingual, administration.

Examples of compressing equipment suitable for the preparing of the tablets of the present invention are rotary presses provided by Elizabeth-Hata International, US (HT series), Courtoy NV, BE (R090F, R100M, R190FT, R290FT, R292F and R233 series), Vector Corp., US (2000, 200 and Magna series), Fette GmbH, DE (Hightech, Medium, Special and WIP series), Manesty, UK (Xpress, Diamond and Value series) and Kilian & Co. GmbH, DE (S, T, E, RX and KTS series).

The composition typically comprises desmopressin acetate in an amount of from 20 to 600 µg per unit of solid dosage form. As an example, a typical tablet containing 100 µg of desmopressin acetate is white, convex and oval (6.7 x 9.5 mm) with a thickness of 3-4 mm and a weight of 200 mg. As another example, a tablet containing 200 µg of desmopressin acetate is white, round (8 mm diameter) and convex with a thickness of 374 mm and a weight of 200 mg.

In a preferred embodiment, each unit of solid dosage form has a hardness of at least 49 N typically with 69 N as the practical upper limit. The hardness test for tablets is performed by measuring the force needed to disrupt the tablets by crushing, using a conventional tablet hardness tester.

To illustrate the present invention in more detail, the following example is provided. It shall not be construed as a limitation of how the invention may be practised.

### Example

### Example 1: Preparation of tablets of desmopressin acetate with varying content of hydrogen peroxide (H₂O₂)

Lactose (478 g, Pharmatose® 150M) and potato starch (308 g, Pharma M20) are weighed separately, mixed, and sieved through a 1 mm mesh size sieve, and then mixed again under low shear conditions. A granulation liquid consisting of water (40.00 g) and ethanol (120.00 g) is prepared, to which desmopressin acetate (0.80 g; provided by PolyPeptide Laboratories AB, Sweden) and PVP (7.36 g; Kollidon® 25; provided by BASF GmbH, Germany) are added.

The granulation liquid is then slowly (over 1 minute) added to the lactose/starch mixture under mixing that is continued for 10 minutes. After sieving (1.4 mm), placement on stainless steel trays, and drying for 30 minutes at ambient temperature followed by at least 4 hours at 45°C, the dried granulate is weighed and sieved (1 mm). Silica (2.40 g, Aerosil® 200 VV Pharma; 1.0 mm sieved) is then added to the granulate under low shear mixing, followed by magnesium stearate (4.00 g, 1.0 mm sieved; provided by Peter Greven, the Netherlands).

The resulting granulate is immediately compressed to tablets using Korsch XL 100 equipment with gravity feeder operating at 40 rpm. The punches and dies are of 8 mm dimension producing convex tablets without score. Each tablet produced weighs about 200 mg, has a hardness of about 50 N and contains H₂O₂ at a level of about 1.5 ppm (the H₂O₂ content stems from the potato starch used).

In total five series of tablets (4000 tablets for each series) are analogously prepared in the above manner with a final H₂O₂ content of 1.7, 4.9, 8.3, 15.6 and 38.7 ppm, respectively. The H₂O₂, where added, is added last to the said granulation liquid in amounts of 0.067, 0.133, 0.267 and 0.667 g (6% aqueous solution), respectively. The volume of water is reduced to the same extent so the total volume of water and 6% hydrogen peroxide is 40.00

The resulting tablets are subjected to a stability study and thereby stored in closed bottles at three different conditions: 25°C at a relative humidity of 60%; 40°C/ambient humidity and 50°C/ambient humidity in climate chambers. The tablet content and purity of desmopressin is monitored over time utilising conventional ultra-violet (UV) spectroscopy detection at 220 nm and liquid chromatography (LC; LiChrospher RP-18, 5 µm, 125 x 4 mm column, mobile phase 0.067 M phosphate buffer pH 7.0/acetonitrile, flow 1.5 ml/min, ambient temperature). Both isocratic and gradient methods are utilised in the LC analysis. The start content of desmopressin is set at 100% at 0 months, i.e. at the start of the stability study.

The content of H₂O₂ over time is determined with a peroxidase/4-aminoantipyrine-chromotropic acid system which turns blue when oxidised. The absorbance is measured at 600 nm wavelength. See Meiattini, F. "Methods of Enzymatic Analysis", vol. 7, pp 566-571 (1985).

The results show that H₂O₂-levels of about 16 ppm, or above, in the tablets at 0 months confer a subsequent degradation rate of desmopressin unsuitably high for storage of a pharmaceutical product.

## Claims

1. A pharmaceutical composition as a solid dosage form comprising desmopressin, or a pharmaceutically acceptable salt thereof, as a therapeutically active ingredient together with a pharmaceutically acceptable excipient, diluent or carrier, or mixture thereof, wherein said pharmaceutical composition comprises silica and starch and wherein the level of oxidising agent is equal to or less than 15 parts per million by weight of said pharmaceutical composition.

2. The pharmaceutical composition according to claim 1 wherein the level of oxidising agent is from 0.01 to less than 5 parts per million by weight of said pharmaceutical composition.

3. The pharmaceutical composition according to claim 2 wherein the level of oxidising agent is from 0.01 to equal to or less than 3 parts per million by weight of said pharmaceutical composition.

4. The pharmaceutical composition according to claim 3 wherein the level of oxidising agent is from 0.01 to equal to or less than 1 part per million by weight of said pharmaceutical composition.

5. The pharmaceutical composition according to any one of claims 1-4, wherein said oxidising agent is a peroxide, such as hydrogen peroxide, or a mixture of peroxides.

6. The pharmaceutical composition according to any one of claims 1-5 which contains silica in an amount of from 0.1 to 1.0, preferably from 0.2 to 0.5, percent by weight of said pharmaceutical composition.

7. The pharmaceutical composition according to any one of claims 1-6 which is composed of a compressed granulate.

8. The pharmaceutical composition according to any of claims 1-7, wherein said starch is potato starch.

9. The pharmaceutical composition according to any one of claims 1-8, wherein the total combined amount of said excipient, diluent or carrier is from 5 to 98, preferably from 50 to 98, percent by weight of said pharmaceutical composition.

10. The pharmaceutical composition according to any one of claims 1-9 which is a perorally available tablet that is optionally adapted for oral, preferably buccal and/or sublingual, administration.

11. The pharmaceutical composition according to any one of claims 1-10 which comprises desmopressin acetate in an amount of from 0.020 to 0.600 mg per unit of solid dosage form.

12. The pharmaceutical composition according to any one of claims 1-11 which is free from preservative.

13. Use of starch containing oxidising agent at a level of equal to or less than 40 parts per million in the preparation of a pharmaceutical composition as a solid dosage form which comprises desmopressin or a pharmaceutically acceptable salt thereof as a therapeutically active ingredient and silica.

14. The use according to claim 13 wherein the starch is potato starch.

15. The use according to claim 13 or 14 wherein the starch contains oxidising agent at a level of from 0.03 to equal to or less than 8 parts per million.

## Patentansprüche

1. Pharmazeutische Zusammensetzung als feste Arzneiform, die Desmopressin oder ein pharmazeutisch akzeptables Salz davon als therapeutisch wirksamen Bestandteil zusammen mit einem pharmazeutisch akzeptablen Exzipienten, Verdünnungsmittel oder Träger oder einer Mischung daraus umfaßt, worin die pharmazeutische Zusammensetzung Kieselerde und Stärke umfaßt und worin der Gehalt an Oxidationsmittel gleich oder weniger als 15 Gew.Teile pro Million der pharmazeutischen Zusammensetzung ist.

2. Pharmazeutische Zusammensetzung gemäß Anspruch 1, worin der Gehalt an Oxidationsmittel 0,01 bis weniger als 5 Gew.Teile pro Million der pharmazeutischen Zusammensetzung ist.

3. Pharmazeutische Zusammensetzung gemäß Anspruch 2, worin der Gehalt an Oxidationsmittel 0,01 bis gleich oder weniger als 3 Gew.Teile pro Million der pharmazeutischen Zusammensetzung ist.

4. Pharmazeutische Zusammensetzung gemäß Anspruch 3, worin der Gehalt an Oxidationsmittel 0,01 bis gleich oder weniger als 1 Gew.Teil pro Million der pharmazeutischen Zusammensetzung ist.

5. Pharmazeutische Zusammensetzung gemäß einem der Ansprüche 1 bis 4, worin das Oxidationsmittel ein Peroxid, wie Wasserstoffperoxid, oder eine Mischung von Peroxiden ist.

6. Pharmazeutische Zusammensetzung gemäß einem der Ansprüche 1 bis 5, die Kieselerde in einer Menge von 0,1 bis 1,0, bevorzugt 0,2 bis 0,5 Gew.% der pharmazeutischen Zusammensetzung enthält.

7. Pharmazeutische Zusammensetzung gemäß einem der Ansprüche 1 bis 6, die aus einem verpreßten Granulat zusammengesetzt ist.

8. Pharmazeutische Zusammensetzung gemäß einem der Ansprüche 1 bis 7, worin die Stärke Kartoffelstärke ist.

9. Pharmazeutische Zusammensetzung gemäß einem der Ansprüche 1 bis 8, worin die kombinierte Gesamtmenge aus dem Exzipienten, Verdünnungsmittel oder Träger 5 bis 98, bevorzugt 50 bis 98 Gew.% der pharmazeutischen Zusammensetzung ist.

10. Pharmazeutische Zusammensetzung gemäß einem der Ansprüche 1 bis 9, die eine peroral verfügbare Tablette ist, die gegebenenfalls zur oralen, bevorzugt bukkalen und/oder sublingualen, Verabreichung angepaßt ist.

11. Pharmazeutische Zusammensetzung gemäß einem der Ansprüche 1 bis 10, die Desmopressinacetat in einer Menge von 0,020 bis 0,600 mg pro Einheit der festen Arzneiform umfaßt.

12. Pharmazeutische Zusammensetzung gemäß einem der Ansprüche 1 bis 11, die frei von Konservierungsmittel ist.

13. Verwendung von Stärke, die Oxidationsmittel in einem Gehalt von gleich oder weniger als 40 Gew.Teilen pro Million enthält, in der Herstellung einer pharmazeutischen Zusammensetzung als feste Arzneiform, die Desmopressin oder ein pharmazeutisch akzeptables Salz davon als therapeutisch wirksamen Bestandteil und Kieselerde umfasst.

14. Verwendung gemäß Anspruch 13, worin die Stärke Kartoffelstärke ist.

15. Verwendung gemäß Anspruch 13 oder 14, worin die Stärke Oxidationsmittel in einem Gehalt von 0,03 bis gleich oder weniger als 8 Teilen pro Million enthält.

## Revendications

1. Composition pharmaceutique comme une forme galénique solide comprenant de la desmopressine, ou un sel pharmaceutiquement acceptable de celle-ci, comme un ingrédient thérapeutiquement actif conjointement avec un excipient, un diluant ou un porteur, ou un mélange de ceux-ci, pharmaceutiquement acceptable, où ladite composition pharmaceutique comprend de la silice et de l'amidon et où le niveau d'agent d'oxydation est inférieur ou égal à 15 parties par million en poids de ladite composition pharmaceutique.

2. Composition pharmaceutique selon la revendication 1 dans laquelle le niveau d'agent d'oxydation est de 0,01 à moins de 5 parties par million en poids de ladite composition pharmaceutique.

3. Composition pharmaceutique selon la revendication 2 dans laquelle le niveau d'agent d'oxydation est de 0,01 jusqu'à 3 parties par million en poids ou moins de ladite composition pharmaceutique.

4. Composition pharmaceutique selon la revendication 3 dans laquelle le niveau d'agent d'oxydation est de 0,01 jusqu'à 1 partie par million en poids ou moins de ladite composition pharmaceutique.

5. Composition pharmaceutique selon l'une quelconque des revendications 1-4, dans laquelle ledit agent d'oxydation est un peroxyde, tel que du peroxyde d'hydrogène, ou un mélange de peroxydes.

6. Composition pharmaceutique selon l'une quelconque des revendications 1-5 qui contient de la silice en une quantité allant de 0,1 à 1,0, de préférence 0,2 à 0,5, pourcent en poids de ladite composition pharmaceutique.

7. Composition pharmaceutique selon l'une quelconque des revendications 1-6 qui est composée de granulé compressé.

8. Composition pharmaceutique selon l'une quelconque des revendications 1-7, dans laquelle ledit amidon est de l'amidon de pomme de terre.

9. Composition pharmaceutique selon l'une quelconque des revendications 1-8, dans laquelle la quantité totale combinée dudit excipient, diluant ou porteur va de 5 à 98, de préférence de 50 à 98, pourcent en poids de ladite composition pharmaceutique.

10. Composition pharmaceutique selon l'une quelconque des revendications 1-9 qui est une tablette perorale qui est optionnellement adaptée pour être administrée par voie orale, de préférence par voie buccale et/ou sublingual.

11. Composition pharmaceutique selon l'une quelconque des revendicationsl-10 qui comprend de l'acétate de desmopressine en une quantité allant de 0,020 à 0,600 mg par unité de forme galénique solide.

12. Composition pharmaceutique selon l'une quelconque des revendications 1-11 qui ne comporte pas d'agents de conservation.

13. Usage d'amidon contenant un agent d'oxydation à un niveau inférieur ou égal à 40 parties par million dans la préparation d'une composition pharmaceutique comme une forme galénique solide qui comprend de la desmopressine ou un sel pharmaceutiquement acceptable de celle-ci comme un ingrédient thérapeutiquement actif et de la silice.

14. Usage selon la revendication 13 dans lequel l'amidon est de l'amidon de pomme de terre.

15. Usage selon la revendication 13 ou 14 dans lequel l'amidon contient un agent d'oxydation à un niveau allant de 0,03 jusqu'à 8 parties par million ou moins.
